(19) Europäisches Patentamt
European Patent Office
Office européen des brevets

(11) Publication number: **0 015 639**
**A2**

# (12) EUROPEAN PATENT APPLICATION

(21) Application number: 80300202.1

(22) Date of filing: 22.01.80

(51) Int. Cl.³: C 07 D 249/08, A 01 N 43/64

(30) Priority: 09.02.79 GB 7904755
04.07.79 GB 7923345

(43) Date of publication of application: 17.09.80
Bulletin 80/19

(84) Designated Contracting States: AT BE CH DE FR GB IT LU NL SE

(71) Applicant: **IMPERIAL CHEMICAL INDUSTRIES LIMITED, Imperial Chemical House Millbank, London SW1P 3JF (GB)**

(72) Inventor: **Sugavanam, Balasubramanyan, 11 Kiln Ride, Wokingham, Berkshire (GB)**

(74) Representative: **Downes, John Edward et al, Imperial Chemical Industries Limited Legal Department: Patents Thames House North Millbank, London SW1P 4QG (GB)**

(54) **Enantiomers of triazole compounds, a process for preparing them, their use as plant fungicides and growth regulating agents and compositions containing them.**

(57) An enantiomer of a compound of formula:

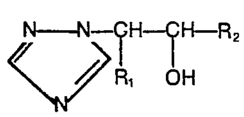

wherein $R_1$ is optionally substituted benzyl; $R_2$ is alkyl; or its esters, salts and metal complexes, the enantiomer being substantially optically pure; the enantiomers are useful as plant fungicides and growth regulating agents.

EP 0 015 639 A2

# ENANTIOMERS OF TRIAZOLE COMPOUNDS, A PROCESS FOR PREPARING THEM, THEIR USE AS PLANT FUNGICIDES AND GROWTH REGULATING AGENTS AND COMPOSITIONS CONTAINING THEM

This invention relates to enantiomers of certain triazole derivatives, to a process for obtaining these enantiomers, to fungicidal and plant growth regulating compositions containing them, to a method of combating fungal infections in, regulating the growth of plants using them.

British Patent Application No. 34590/76 which corresponds to Dutch Patent Application No. 77019197 (the disclosure of which Applications is incorporated herein by reference), discloses *inter alia* compounds of general formula (I):

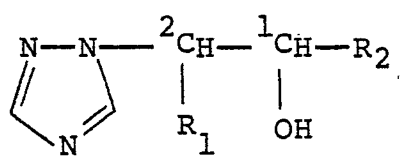

wherein $R_1$ is benzyl optionally ring substituted with up to three substituents selected from the class consisting of halogen, alkyl and alkoxy, and $R_2$ is $C_{1-6}$ alkyl, and their esters, acid addition salts and metal complexes. The compounds are stated to have plant fungicidal and plant growth regulating activities.

It will be appreciated that these compounds contain two chiral centres. This means that the compounds can exist in four optical isomeric forms. Two of these isomers are enantiomers (i.e. mirror images) of each other, as are the other two isomers. One pair of enantiomers are diastereoisomers of the other pair of enantiomers. Using the conventional nomenclature system for enantiomers having known absolute configurations, the four enantiomers can be designated as:

| | | |
|---|---|---|
| (a) | (R) $C^2$ | (R) $C^1$ |
| (b) | (S) $C^2$ | (S) $C^1$ |
| (c) | (R) $C^2$ | (S) $C^1$ |
| (d) | (S) $C^2$ | (R) $C^1$ |

Isomers (a) and (b) are enantiomers of each other and they are diastereoisomers of isomers (c) and (d) which are also enantiomers of each other.

British Patent Application No. 34590/76 discloses the diastereoisomers of the compound wherein $R_1$ is parachlorobenzyl and $R_2$ is t-butyl - see Compounds 1 and 5 of Table I in British Application No. 34590/76. Compound 1 is a racemic mixture of an enantiomer wherein both the carbon carrying the triazole ring and the carbon carrying the hydroxy are in the R-configuration, while Compound 5 is a racemic mixture of an enantiomer wherein the carbon carrying the triazole ring is in the R-configuration and the carbon carrying the hydroxy group is in the S-configuration and an enantiomer which is its mirror image; in fact, Compound 1 is a racemic mixture of enantiomers (a) and (b) and compound 5 is a racemic mixture of enantiomers (c) and (d). Compounds 1 and 5 were found to have approximately the same activities as plant fungicides and plant growth regulating agents, but compound 1 is generally more active than compound 5.

We have now found that when the two enantiomers of a diastereoisomeric mixture of this type are separated there can be a significant separation of the fungicidal and plant growth regulating activities between the two enantiomers. For example, we have resolved compound 1 (which is a racemic mixture) into its enantiomers and have found that one of them, the (+) enantiomer, which is now known from X-ray analysis to be enantiomer (a), has high fungicidal activity but low plant growth regulating activity. The other isomer, the (-) enantiomer [which is enantiomer (b)], has not yet been obtained completely optically pure; however, testing of an optically slightly impure sample has suggested that

it may have lower fungicidal activity but higher plant growth regulating activity than the racemic mixture and the (+) enantiomer. Further, compound 9 (which is also a racemic mixture) has been resolved into its enantiomers and we have found that one of them, the (+) enantiomer [which is enantiomer (a)], has higher fungicidal but lower plant growth regulating activity than the racemic mixture; in addition, the (+) enantiomer, unlike the racemic mixture, is volatile enough to be active in the vapour phase against fungi on the plants.

The invention therefore provides a compound which is an enantiomer of a compound of general formula (I) or an ester, acid addition salt or metal complex of such an enantiomer, the enantiomer being substantially optically pure (i.e. it is associated with no more than 10% of the other enantiomers). The optical purity of an enantiomer can be established by nuclear magnetic resonance techniques using a chiral shift reagent.

The alkyl groups, which can be straight or branched chain, preferably have 1 to 5 carbon atoms; examples are methyl, ethyl, propyl (n- or i-propyl) and butyl (n-, sec-, i- or t-butyl).

Suitable substituents on the phenyl moiety of the benzyl are halogen (e.g. fluorine, chlorine, bromine or iodine), $C_{1-4}$ alkyl [e.g. methyl, ethyl, propyl (n- or i-propyl) and butyl (n-, sec, i- or t-butyl)], and $C_{1-4}$ alkoxy (e.g. methoxy, ethoxy, propoxy or butoxy).

Examples of suitable benzyl groups are benzyl itself, chlorobenzyl (for example o-, m- or p-chlorobenzyl), dichlorobenzyl (e.g. 3,4-, 2,4-, 3,5- or 2,6-dichlorobenzyl), trichlorobenzyl (e.g. 2,3,6- or 2,4,5-trichlorobenzyl), bromobenzyl (e.g. o-, m- or p-bromobenzyl), dibromobenzyl (e.g. 2,4-dibromobenzyl), fluorobenzyl (e.g. o-, m- or p-fluorobenzyl), difluorbenzyl (e.g. 2,4- or 3,4-difluorobenzyl), iodobenzyl (e.g. o-iodobenzyl), methylbenzyl (e.g. o-, m- or p-methylbenzyl), dimethylbenzyl (e.g. 2,6-, 2,5- or 3,4-dimethylbenzyl), ethylbenzyl (e.g. p-ethylbenzyl), propylbenzyl (e.g. p-i-propylbenzyl), butylbenzyl (e.g. p-t-butylbenzyl), methoxybenzyl (e.g. o-, m- or p-methoxybenzyl), dimethoxybenzyl (e.g. 2,4-, 3,4- or 3,5-dimethoxybenzyl), ethoxybenzyl (e.g. o-, m- or p-ethoxybenzyl), propoxybenzyl (e.g. p-i-propoxybenzyl or p-n-propoxybenzyl), butoxybenzyl (e.g. o-, m- or p-i-butoxybenzyl), chlorofluorobenzyl (e.g. 2-fluoro-4-chlorobenzyl, 2-chloro-6-fluorobenzyl or 2-chloro-4-fluorobenzyl), fluorobromobenzyl (e.g. 2-fluoro-4-bromobenzyl), methoxychlorobenzyl (e.g. 3-chloro-4-methoxybenzyl), methoxybromobenzyl (e.g. 2-methoxy-5-bromobenzyl or 3-bromo-4-methoxybenzyl), ethoxychlorobenzyl (e.g. 4-ethoxy-3-chlorobenzyl) and ethoxybromobenzyl (e.g. 4-ethoxy-3-bromobenzyl).

A preferred class of compounds are those wherein $R_1$ is benzyl, chlorobenzyl, dichlorobenzyl, trichlorobenzyl, bromobenzyl, dibromobenzyl, fluorobenzyl, difluorobenzyl, iodobenzyl, methylbenzyl, dimethylbenzyl, ethylbenzyl, propylbenzyl, butylbenzyl, methoxybenzyl, ethoxybenzyl, chlorofluorobenzyl, fluorobromobenzyl, and methoxybromobenzyl, and $R_2$ is propyl or butyl.

Particularly preferred are those compounds wherein $R_1$ is benzyl, o-, m- or p-chlorobenzyl, 2,4-, 3,4- or 2,6-dichlorobenzyl, 2,4,5- or 2,3,6-trichlorobenzyl, o-, m- or p-bromobenzyl, 2,4-dibromobenzyl, o-, m- or p-fluorobenzyl, 2,4-difluorobenzyl, o-iodobenzyl, o-, m- or p-methylbenzyl, 2,5- or 3,4-dimethylbenzyl, p-ethylbenzyl, p-i-propylbenzyl, p-t-butylbenzyl, o-, m-or p-methoxybenzyl, o-, m- or p-ethoxybenzyl, 2-chloro-4-fluorobenzyl, 2-fluoro-4-chlorobenzyl, 2-chloro-6-fluorobenzyl, 2-fluoro-4-bromobenzyl or 3-bromo-4-methoxybenzyl, and $R_2$ is i-propyl, i-butyl or t-butyl.

Suitable salts are salts with inorganic or organic acids, e.g. hydrochloric, nitric, sulphuric, toluenesulphonic, acetic or oxalic acid. The esters are suitably alkanoates (e.g. acetates).

The metal complex is suitably one including copper, zinc, manganese or iron. It preferably has the general formula:

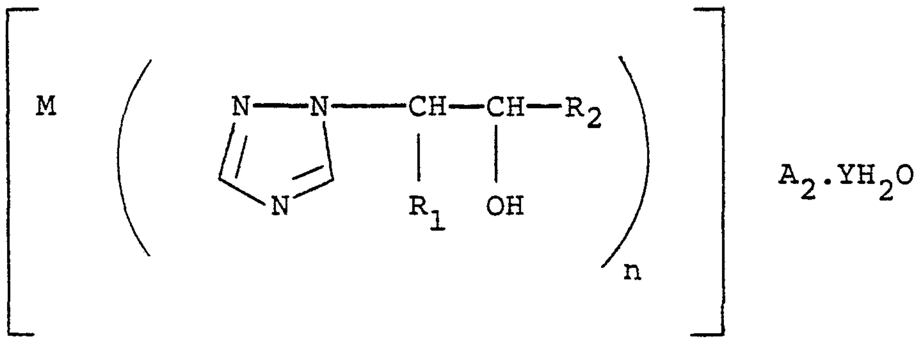

wherein $R_1$ and $R_2$ are as defined above, M is a metal, A is an anion (e.g. a chloride, bromide, iodide, nitrate, sulphate or phosphate anion), n is 2 or 4, and Y is O or an integer of 1 to 12.

Table I gives specific examples of compounds of general formula (I) which are suitable for resolution into enantiomers.

TABLE I

| NO | $R_1$ | $R_2$ | MELTING (OR BOILING) POINT °C |
|---|---|---|---|
| 1* | p-Cl-$C_6H_4CH_2$- | t-Bu | 162-164° |
| 2 | $C_6H_5CH_2$- | t-Bu | 91-93° |
| 3 | p-F-$C_6H_4CH_2$- | t-Bu | 137-142° |
| 4[x] | p-Cl-$C_6H_4CH_2$ | t-Bu | 109-111° |
| 5* | p-Cl-$C_6H_4CH_2$- | t-Bu | 133-134° |
| 6 | p-Cl-$C_6H_4CH_2$- | t-Bu | 179-181° |
| 7 | 3,4-diCl-$C_6H_3CH_2$ | t-Bu | 186-188° |
| 8 | o-F-$C_6H_4CH_2$ | t-Bu | 100-102° |
| 9 | 2,4-diCl-$C_6H_3CH_2$ | t-Bu | 140-143° |
| 10 | o-Cl-$C_6H_4CH_2$ | t-Bu | 100-102° |

TABLE I Continued..

| No | $R_1$ | $R_2$ | MELTING (OR BOILING) POINT °C |
|---|---|---|---|
| 11 | p-Br-$C_6H_4CH_2$ | t-Bu | 181-183° |
| 12 | m-F-$C_6H_4CH_2$ | t-Bu | 110-113° |
| 13 | m-Br-$C_6H_4CH_2$ | t-Bu | 133-136° |
| 14 | 2,4-diCl-$C_6H_3CH_2$ | i-Pr | 127-130° |
| 15 | p-Cl-$C_6H_4CH_2$ | i-Pr | 100-103° |
| 16+ | p-Cl-$C_6H_4CH_2$ | t-Bu | 138-140° |
| 17° | p-F-$C_6H_4CH_2$ | i-Pr | 74-78° |
| 18 | 2,6-diCl-$C_6H_3CH_2$ | t-Bu | 151-154° |
| 19° | 2-Cl-4-F-$C_6H_3CH_2$ | t-Bu | 137-140° |
| 20° | o-F-$C_6H_4CH_2$ | i-Pr | 122-127° |

TABLE I Continued..

| NO | $R_1$ | $R_2$ | MELTING (OR BOILING) POINT $^oC$ |
|---|---|---|---|
| 21 | 2,4,5-triCl-$C_6H_2CH_2$ | t-Bu | 188-192$^o$ |
| 22 | 2,3,6-triCl-$C_6H_2CH_2$ | t-Bu | 168-172$^o$ |
| 23 | 2-F-4-Cl-$C_6H_3CH_2$ | t-Bu | 150-153$^o$ |
| 24 | 2,4-diF-$C_6H_3CH_2$ | t-Bu | 111-114$^o$ |
| 25 | 2-F-4-Br-$C_6H_3CH_2$ | t-Bu | 171-174$^o$ |
| 26 | 2,4-diBr-$C_6H_3CH_2$ | t-Bu | 157-160$^o$ |
| 27 | o-MeO-$C_6H_4CH_2$ | t-Bu | 141-144$^o$ |
| 28 | o-Me-$C_6H_4CH_2$ | t-Bu | 123-125$^o$ |
| 29 | p-Me-$C_6H_4CH_2$ | t-Bu | 144-146$^o$ |
| 30 | 2,5-diMe-$C_6H_3CH_2$ | t-Bu | 114-117$^o$ |

TABLE I Continued..

| NO. | $R_1$ | $R_2$ | MELTING (OR BOILING) POINT $^oC$ |
|---|---|---|---|
| 31 | m-Cl-$C_6H_4CH_2$ | t-Bu | 127-129° |
| 32 | p-MeO-$C_6H_3$-$CH_2$ | t-Bu | 116-118° |
| 33 | 2-MeO-5-Br-$C_6H_3CH_2$ | t-Bu | 184-186° |
| 34 | p-EtO-$C_6H_4CH_2$ | t-Bu | 116-117° |
| 35 | o-EtO-$C_6H_4CH_2$ | t-Bu | 152-154° |
| 36 | 2,4-diCl-$C_6H_3CH_2$ | t-Bu | 197-199° |
| 37 | o-Br-$C_6H_4CH_2$ | t-Bu | 113-114.5° |
| 38 | 3-Br-4-MeO-$C_6H_3CH_2$ | t-Bu | 163° |
| 39 | p-Cl-$C_6H_4CH_2$ | i-Bu | 45-50° |
| 40 | 2,4-diCl-$C_6H_3CH_2$ | i-Bu | 119-123° |

TABLE I Continued..

| NO | $R_1$ | $R_2$ | MELTING (OR BOILING) POINT $^oC$ |
|---|---|---|---|
| 41 | o-I-$C_6H_4CH_2$ | t-Bu | 117-119$^o$ |
| 42 | p-(t-Bu)-$C_6H_4CH_2$ | t-Bu | 90$^o$ |
| 43 | 3-Cl-4-MeO-$C_6H_3CH_2$ | t-Bu | 148$^o$ |
| 44 | 2-Cl-6-F-$C_6H_3CH_2$ | t-Bu | 120-122$^o$ |
| 45 | p-F-$C_6H_4CH_2$ | i-Bu | |
| 46 | m-Me-$C_6H_4CH_2$ | t-Bu | 124$^o$ |
| 47 | p-Et-$C_6H_4CH_2$ | t-Bu | 89$^o$ |
| 48 | 2-Cl-4-F-$C_6H_3CH_2$ | i-Bu | 94-97$^o$ |
| 49 | $C_6H_5CH_2$ | i-Bu | oil |
| 50 | m-EtO-$C_6H_4CH_2$ | t-Bu | 106-108$^o$ |

TABLE I Continued..

| NO | $R_1$ | $R_2$ | MELTING (OR BOILING) POINT $^oC$ |
|---|---|---|---|
| 51 | 3,4-diMe-$C_6H_3CH_2$ | t-Bu | $141^o$ |
| 52 | p-(i-Pr)-$C_6H_4CH_2$ | t-Bu | |
| 53 | p-Me-$C_6H_4CH_2$ | i-Bu | $70-73^o$ |
| 54 | o-F-$C_6H_4CH_2$ | i-Bu | $64-68^o$ |
| $55^x$ | 2,4-diCl-$C_6H_3CH_2$ | t-Bu | $126-128^o$ |

*Compounds 1 and 5 are diastereoisomers of each other as are Compounds 9 and 36. Each of Compounds 1 and 9 is a racemic mixture of an enantiomer wherein both the carbon carrying the triazole ring and the carbon carrying the hydroxy group are in the R-configuration and an enantiomer which is its mirror image. Each of Compounds 5 and 36 is a racemic mixture of an enantiomer wherein the carbon carrying the triazole ring is in the R-configuration and the carbon carrying the hydroxy group is in the S-configuration and an enantiomer which is its mirror image.

+Compound 16 is in the form of a copper complex believed to have the structure

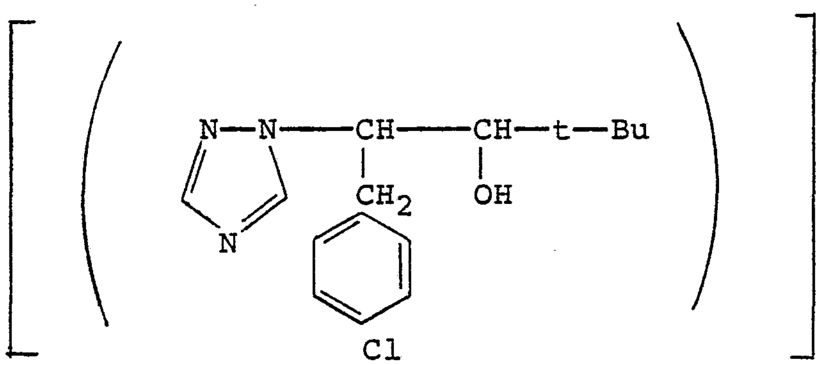

°Nuclear magnetic resonance studies have shown that Compounds 17, 19 and 20 are each in the form of a mixture of diastereoisomers. The weight ratios of the two diastereoisomers in each case are as follows:

| Compound | Weight ratio |
|---|---|
| 17 | 9:1 |
| 19 | 7:1 |
| 20 | 4:1 |

ˣCompound 4 is the acetate of Compound 1 and Compound 55 is the acetate of Compound 9.

There now follows a discussion of the preparation the diastereoisomeric mixtures suitable for resolution into enantiomers.

The racemic mixtures of isomers 1 and 2 of the compound of general formula (I), or a salt thereof, can be prepared by reducing, preferably at 0 to 100°C and for 1 to 12 hours, a compound of general formula (II):

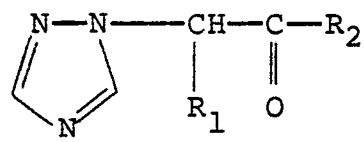

wherein Y, $R_1$ and $R_2$ are as defined above, or a salt thereof. Suitable reducing agents are sodium borohydride, lithium aluminium hydride or aluminium isopropoxide. If desired, catalytic hydrogenation using a suitable metal catalyst can be used. When the compound of general formula (II) is a sterically hindered ketone, a Grignard reagent, for example butylmagnesium halide (e.g. bromide or iodide) can be used as the reducing agent; when a reagent such as a butyl-magnesium halide is used, single diastereoisomers are often produced.

The reduction can be performed by dissolving the reactants in a solvent such as diethyl ether or tetrahydrofuran (for lithium aluminium hydride reduction) or hydroxylic solvents (for sodium borohydride reduction). The reaction temperature will depend on the reactants and solvent; but generally the reaction mexture is heated under reflux. After the reaction, the product can be isolated by extraction into a convenient solvent after acidification with dilute mineral acid. On removal of the solvent in vacuo, the product may be crystallised from a convenient solvent.

The racemic mixture of isomers 3 and 4 of the compound of general formula (I), or a salt thereof, can be prepared by reacting, preferably at 15 to 80°C and for 6 to 12 hours, a compound of general formula (II) or a salt thereof with the appropriate Grignard reagent

e.g. a methyl or alkenyl magnesium halide such as methyl or allyl magnesium bromide or iodide. This reaction can be performed by methods known in the art.

Details of the preparation of the compounds of general formula (II) can be found in British Patent Application No. 34590/76.

The salts, metal complexes and esters of the compounds of general formula (I) can be prepared from the latter in known manner. For example, the complexes can be made by reacting the uncomplexed compound with a metal salt in a suitable solvent. Further, the substituent on the ring of the benzyl in the compound of general formula (I) can be changed by methods known in the art.

As indicated above the compounds of general formula (I) are prepared by the above reactions in the form of racemic mixtures. The resolution of these mixtures into the constituent enantiomers can be performed by known methods. Examples of these methods are (1) forming the diastereoisomeric salts or esters of the compound of general formula (I) with an optically active acid (e.g. camphor sulphonic acid), separating the isomeric salts or esters and converting the separated isomeric salts or esters into the enantiomers of the compound of general formula (I); (2) forming the diastereoisomeric carbamates of the compound of general formula (I) by reacting a haloformate (e.g. chloroformate) of the latter with an optically active amine (e.g. α-methylbenzylamine), separating the isomeric carbamates, and converting the separated isomeric carbamates into the enantiomers of the compound of general formula (I); or (3) resolving the mixtures using enantioselective crystallisation techniques (Leigh, Chemistry and Industry, 1970, pages 1016-1017, and <u>ibid</u>, 1977, page 36). The separation of the diastereoisomeric salts, esters and carbamates can be achieved by for example crystallisation techniques or by

high pressure liquid chromatography (HPLC). Alternatively, the enantiomers can be prepared directly from the compound of general formula (II) by stereospecific reduction, for example by biochemical reduction (using for example yeast or Aspergillus niger) or by hydrogenation using chiral catalysts (e.g. a Wilkinson's catalyst) or by reduction with borohydride/amino acid complexes.

The enantiomer compounds of the invention and their esters, salts and metal complexes (hereinafter referred to as "the compounds") can have fungicidal activity particularly against the diseases:

Piricularia oryzae on rice

Puccinia recondita, Puccinia striiformis and other rusts on wheat, Puccinia hordei, Puccinia striiformis and other rusts on barley, and rusts on other hosts e.g. coffee, apples, vegetables and ornamental plants

Plasmopara viticola on vines

Erysiphe graminis (powdery mildew) on barley and wheat and other powdery mildews on various hosts such as Sphaerotheca fuliginea on cucurbits (e.g. cucumber), Podosphaera leucotricha on apples and Uncinula necator on vines

Helminthosporium spp. on cereals

Cercospora arachidicola on peanuts and other Cercospora species on for example sugar beet, bananas and soya beans

Botrytis cinerea (grey mould) on tomatoes, strawberries, vines and other hosts

Phytophthora infestans (late blight) on tomatoes

Venturia inaequalis (scab) on apples

Some of the compounds have also shown a broad range of activities against fungi in vitro. They have activity against various post-harvest diseases on fruit (e.g. Penicillium digatatum and italicum on oranges and Gloeosporium musarum on bananas). Further some of the compounds are active as seed dressings against: Fusarium spp., Septoria spp., Tilletia spp. (i.e. bunt, a seed borne disease of wheat), Ustilago spp., Helminthosporium

spp. on cereals, Rhizoctonia solani on cotton and Corticium sasakii on rice.

The compounds can also have plant growth regulating activities.

The plant growth regulating effects of the compounds are manifested as for example a stunting or dwarfing effect on the vegetative growth of woody and herbaceous mono- and di-cotyledonous plants. Such stunting or dwarfing may be useful, for example, in peanuts, cereals and soya bean where reduction in stem growth may reduce the risk of lodging and may also permit increased amounts of fertiliser to be applied. The stunting of woody species is useful in controlling the growth of undergrowth under power lines etc. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. Examples of suitable grasses are Stenotaphrum secundatum (St. Augustine grass), Cynosurus cristatus, Lolium multiflorum and perenne, Agrostis tenuis, Cynodon dactylon (Bermuda grass), Dactylis glomerata, Festuca spp. (e.g. Festuca rubra) and Poa spp. (e.g. Poa pratense). The compounds may stunt grasses without significant phytotoxic effects and without deleteriously affecting the appearance (particularly the colour) of the grass; this makes such compounds attractive for use on ornamental lawns and on grass verges. They may also have an effect on flower head emergence in for example grasses. The compounds can also stunt weed species present in the grasses; examples of such weed species are sedges (e.g. Cyperus spp.) and dicotyledonous weeds (e.g. daisy, plantain, knotweed, speedwell, thistle, docks and ragwort). The growth of non-crop vegetation (e.g. weeds or cover vegetation) can

be retarded thus assisting in the maintenance of plantation and field crops. In fruit orchards, particularly orchards subject to soil erosion, the presence of grass cover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species may be stunted more than another grass species; this selectivity could be useful for example for improving the quality of a sward by preferential suppression of the growth of undesirable species.

The dwarfing may also be useful in miniaturising ornamental, household, garden and nursery plants (e.g. poinsettias, chrysanthemums, carnations, tulips and daffodils).

As indicated above, the compounds can also be used to stunt woody species. This property can be used to control hedgerows or to shape fruit trees (e.g. apples). Some coniferous trees are not significantly stunted by the compounds so the compounds could be useful in controlling undesirable vegetation in conifer nurseries.

The plant growth regulating effect may (as implied above) manifest itself in an increase in crop yield.

In the potato, vine control in the field and inhibition of sprouting in the store may be possible.

Other plant growth regulating effects caused by the compounds include alteration of leaf angle and promotion of tillering in monocotyledonous plants. The former effect may be useful for example in altering the leaf orientation of, for example, potato crops thereby letting more light into the crops and inducing an increase in phytosynthesis and tuber weight. By increasing tillering in monocotyledonous crops (e.g. rice), the number of

flowering shoots per unit area may be increased thereby increasing the overall grain yield of such crops. In grass swards an increase in tillering could lead to a denser sward which may result in increased resilience in wear.

The treatment of plants with the compounds can lead to the leaves developing a darker green colour.

The compounds may inhibit, or at least delay, the flowering of sugar beet and thereby may increase sugar yield. They may also reduce the size of sugar beet without reducing significantly the sugar yield thereby enabling an increase in planting density to be made. Similarly in other root crops (e.g. turnip, swede, mangold, parsnip, beetroot, yam and cassava) it may be possible to increase the planting density.

The compounds could be useful in restricting the vegetative growth of cotton thereby leading to an increase in cotton yield.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below the snow cover during the cold weather. Further the compounds may cause drought or cold resistance in certain plants.

When applied as seed treatments at low rates the compounds can have a growth stimulating effect on plants.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.1 to 15, preferably 0.1 to 5, kg per hectare is used. However, on certain plants even

application rates within these ranges may give undesired phytotoxic effects. Routine tests may be necessary to determine the best rate of application of a specific compound for any specific purpose for which it is suitable.

The compounds may be used as such for fungicidal or plant growth regulating purposes but are more conveniently formulated into compositions for such usage. The invention thus provides also a fungicidal or plant growth regulating composition comprising a compound which is an enantiomer of the invention or an ester, salt or complex thereof, and a carrier or diluent.

The invention also provides a method of combating fungal diseases in a plant, which method comprises applying to the plant, to seed of the plant or to the locus of the plant or seed a compound which is an enantiomer of the invention or an ester, salt or complex thereof.

It also provides a method of regulating the growth of a plant, which method comprises applying to the plant, to seed of the plant or to the locus of the plant or seed a compound which is an enantiomer of the invention or an ester, salt or complex thereof as hereinbefore defined.

The compounds can be applied in a number of ways, for example they can be formulated or unformulated, directly to the foliage of a plant, to seeds or to other medium in which plants are growing or are to be planted, or they can be sprayed on, dusted on or applied as a cream or paste formulation, or they can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compounds are preferably used for agricultural

and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (e.g. 2-ethoxyethanol and 2-butoxyethanol).

The compositions to be used as sprays may also be in the form of aerosols wherein the formulation is held in a

container under pressure in the presence of a propellant, e.g. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form.

By including suitable additives, for example additives for improving the distribution, adhesive power and resistance to rain on treated surfaces, the different compositions can be better adapted for various utilities.

The compounds can be used as mixtures with fertilisers (e.g. nitrogen-, potassium- or phosphorus-containing fertilisers). Compositions comprising only granules of fertiliser incorporating, for example coated with, the compound, are preferred. Such granules suitably contain up to 25% by weight of the compound. The invention therefore also provides a fertiliser composition comprising a compound as hereinbefore defined.

The compositions may also be in the form of liquid preparations for use as dips or sprays which are generally aqueous dispersions or emulsions containing the active ingredient in the presence of one or more surfactants e.g. wetting agent(s), dispersing agent(s), emulsifying agent(s) or suspending agent(s). These agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl- and triisopropyl-naphthalene sulphonates).

Suitable non-ionic agents are the condensation products of ethylene oxide with fatty alcohols such as

oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient(s). These concentrates suitably contain organic acids (e.g. alkaryl or aryl sulphonic acids such as xylenesulphonic acid or dodecylbenzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, e.g. compounds having similar or complementary fungicidal or

plant growth regulating activity or compounds having herbicidal or insecticidal activity.

The other fungicidal compound can be for example one which is capable of combating ear diseases of cereals (e.g. wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compounds are imazalil, benomyl, carbendazim (BCM), thiophanate-methyl, captafol, captan, sulphur, thiabendazole, dithiocarbamates, carbathiins, copper oxychloride, triforine, dodemorph, tridemorph, dithianon, pyrazophos, binapacryl, quinomethionate, panoctine, furalaxyl, aluminium tris(ethylphosphonate), DPX3217, ethirimol, dimethirimol, bupirimate, chlorothalonil, Chevron RE20615, vinclozolin, procymidone, iprodione and metaxanine.

The compounds of the invention can be mixed with soil, peat or other rooting media for the protection of plants against seed-borne, soil-borne or foliar fungal diseases.

Suitable insecticides are pirimor, croneton, dimethoate, metasystox and formothion.

The other plant growth regulating compound can be one which controls weeds or seedhead formation, improves the level or longevity of the plant growth regulating activity of the compounds of general formula (I), selectively controls the growth of the less desirable plants (e.g. grasses) or causes the compound of general formula (I) to act faster or slower as a plant growth regulating agent. Some of these other agents will be herbicides. Examples of suitable agents are the gibberellins (e.g. $GA_3$, $GA_4$ or $GA_7$), the auxins (e.g. indoleacetic acid, indolebutyric acid, naphthoxyacetic acid or naphthylacetic acid), the cytokinins (e.g. kinetin, diphenylurea, benzimidazole, benzyladenine or BAP), phenoxyacetic acids (e.g. 2,4-D or MCPA), substituted benzoic acids (e.g. TIBA), morphactins (e.g. chlorfluorecol), maleic hydrazide, glyphosate, glyphosine, long chain fatty alcohols and acids (e.g. Off Shoot O or Off Shoot T), dikegulac, Sustar, Embark, substituted quaternary ammonium and phosphonium compounds (e.g. CCC or Phosfon-D), Ethrel, carbetamide, Racuza, Alar, asulam, abscissic acid, isopyrimol, RH531, hydroxybenzonitriles (e.g. bromoxynil), Avenge, Suffix or Lontrel.

The following Examples illustrate the invention; the temperatures are given in degrees Centigrade ($^{o}$).

EXAMPLE 1

(2R, 3R)-1-(4-chlorophenyl)-4,4-dimethyl-2-(1,2,4-triazol-1-yl)pentan-3-ol (the + enantiomer of Compound 1)

A solution of α-p-chlorobenzyl-α-1,2,4-triazol-1-Yl-pinacolone in methanol (20ml) was treated portionwise with sodium borohydride (0.26g). The reaction mixture was then refluxed for one hour. The solvent was removed in vacuo and hydrochloric acid (1 N; 40ml) was added to the residue. The white precipitate was filtered off, washed with water, dried and crystallised from aqueous ethanol to give the racemic mixture (2R, 3R + 2S, 3S)-(4-chlorophenyl)-4,4-dimethyl-2-(1,2,4-triazol-1-yl)pentan-3-one as a white crystalline solid, m.p. 162-164$^{o}$.

The racemic mixture (9.6g) and camphor sulphonic acid (9.2g) in ethanol/ethyl acetate (1:1; 150ml) were refluxed for 16 hours. The solvent was removed in vacuo and the residue was treated with petroleum ether (60-80$^{o}$)/ethyl acetate (1:1; 250ml). Filtration of the mixture gave a solid (7.8g), m.p. 167$^{o}$, which was crystallised from acetonitrile. The crystals which

crystallised at room temperature were filtered off to give crystals (3.6g), m.p. 168-170°, of the camphor sulphonic acid salt of the + enantiomer. These crystals were mixed with water (25ml), and the mixture treated with ammonia (10ml) and stirred for 2 hours. The solid was filtered off and dried to give the (+) enantiomer (2g), m.p. 145-146°; $[\alpha]_D^{21}$ 96° ($CHCl_3$, 3).

EXAMPLE 2

(2R, 3R)-1-(2,4-dichlorophenyl)-4,4-dimethyl-2-(1,2,4-triazol-1-yl)pentan-3-ol (the + enantiomer of Compound 9)

To a solution of the racemic mixture (2R, 3R + 2S, 3S)-1-(2,4-dichlorophenyl)-4,4-dimethyl-2-(1,2,4-triazol-1-yl)-pentan-3-ol (5.09g) in methylene chloride (200ml) was added 4-dimethylaminopyridine (5.68g) and the mixture was cooled to 0°. To this stirred solution, was added, with stirring, phosgene (20.8g) dissolved in 1,2-dichloroethane (50ml). A white precipitate was formed and the stirring was continued at room temperature for 30 hours. The solid was filtered off and the filtrate was concentrated to 50ml and treated with (-)-α-methylbenzylamine (5.64g). The mixture was left at room temperature for 48 hours. The solvent was removed *in vacuo* and the residual gum was purified by chromatography on silica gel (0.040-0.063 mm), eluting with ethyl acetate. Crystallisation from ethyl acetate/petroleum ether (60-80°) gave the carbamate, m.p. 116.5-118.5°.

The diastereoisomeric carbamate was separated by HPLC using Water Associates-2-Prep.Pak. silica gel cartridges and petroleum ether (40-60°)/methylene chloride/ethanol (87:10:13) as the mobile face. A solution of the carbamate enantiomer (1.2g) so produced in 1,2-dichloroethane (25ml) was treated under an inert

atmosphere with triethylamine (1.36g) dissolved in 1,2-dichloroethane (15ml) followed by trichlorosilane (1.09g) in 1,2-dichloroethane (10ml). The reaction mixture was stirred at room temperature for 72 hours and treated with saturated ammonium chloride solution (10ml) for one hour. The organic layer was washed with saturated ammonium chloride solution and dried ($MgSO_4$), and the solvent was removed in vacuo. The residual gum was purified by preparative thin layer chromatography using silica gel and ethyl acetate as the eluent. The (+) enantiomer was obtained as a solid, m.p. 124-126°; $[\alpha]_D^{21}$ 110° (Ethanol, 0.5).

EXAMPLE 3

This Example shows that the (+) enantiomer of Compound 9 has lower plant growth regulating (PGR) activity on apple seedlings than the racemic mixture which is Compound 9.

Apple seedlings (Red Delicious) growing in 7cm pots of loamless compost were sprayed to maximum retention with the test compounds fromulated as aqueous dispersions. Each treatment was appled to 5 replicate plants. The plants were sprayed twice with a 7 day interval, and misted with deionised water twice per week. After the sprays had dried, the plants were kept in a growth room ('day' conditions: 24°C, 60% RH; 'night' conditions: 17°C, 95% RH; 16 hour day). The PGR activity was assessed 14-16 days after the first spray. The results are shown in Table II.

TABLE II

| Compound | ppm a.i. in spray | PGR activity* | notes |
|---|---|---|---|
| Untreated | - | 0 | No PGR activity |
| Compound 10 | 250 | 3 | Substantial reduction in internode length; darkening of leaves and reduction in leaf size. |
| (+) Enantiomer of Compound 10 | 250 | 1 | Slight stunting and reduction in leaf size. |

* Graded on an arbitrary scale:

0 = no PGR activity

5 = severe PGR activity (plant dead)

EXAMPLE 4

This Example shows that the (+) enantiomer of Compound 1 has lower plant growth regulating activity on apple seedlings than the racemic mixture which is Compound 1.

The technique used was the same as in Example 3 except that the plants were kept in a glasshouse at a temperature of 13-29°C and only 4 replicates were used. The results are shown in Table III.

0015639

TABLE III

| Compound | ppm a.i. in spray | PGR activity* | notes |
|---|---|---|---|
| Untreated | - | 0 | No PGR activity |
| Compound 1 | 250 | 3 | Severe stunting, internode shortening, darkening of leaves, and reduction in leaf size. |
| (+) Enantiomer of Compound 1 | 250 | 1 | Slight stunting and reduction in leaf size. |

* see Example 3.

RFF/ayl
11 Jan 1980

EXAMPLE 5

This Example compares the stem shortening effect of Compound 1 (a racemic mixture) with its + enantiomer.

A wheat cultivar (Kador) was sprayed with test compound in aqueous solution containing the surface active ingredient Synperonic A7. Cycocel was included as a standard (but no Synperonic A7 was included). Plants were sprayed at growth stage 6 (on the Feekes-Large scale), i.e. when one node was detectable and the first six leaves were fully expanded. The volume sprayed was equivalent to a rate of 300 l./ha. The amount of Synperonic A7 in the spray was equivalent to 125g/ha. Stem lengths were measured 29 and 45 days after treatment. The results are shown in Table IV.

TABLE IV

| Compound | ppm a.i. in spray | % increase (+) or decrease (-) in height | |
|---|---|---|---|
| | | 29 DAT | 29 DAT |
| Compound 1 | 125 | -9.4 | -4.5 |
| + enantiomer | 62.5 | +0.8 | +0.7 |
| of Compound 1 | 1.25 | +3.3 | +1.4 |
| | 250 | +0.9 | +0.3 |
| Cycocel | 1700 | -14.3 | -11.7 |
| Untreated | - | 0 | 0 |

The fungicidal activity against cereal rusts and mildews of the + enantiomer has been found to be generally higher than the racemic mixture.

1. An enantiomer of a compound of general formula (I)

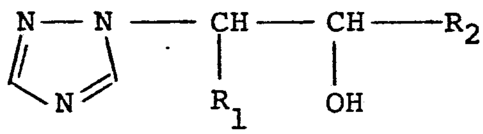

wherein $R_1$ is benzyl optionally ring substituted with up to three substituents selected from the class consisting of halogen, alkyl or alkoxy, and $R_2$ is $C_{1-6}$ alkyl, or an ester, acid addition salt or metal complex thereof, the enantiomer being substantially optically pure.

2. An enantiomer according to claim 1 characterised in that in the compound of general formula (I), $R_1$ is mono- or di-halobenzyl and $R_2$ is butyl.

3. An enantiomer according to claim 2 characterised in that in the compound of general formula (I), $R_1$ is mono- or di-halobenzyl and $R_2$ is t-butyl.

4. An enantiomer according to claim 3 which is (2R,3R) -1-(4-chlorophenyl)-4,4-dimethyl-2-(1,2,4-triazol-1-yl)pentan-3-ol.

5. An enantiomer according to claim 3 which is (2R,3R) -1-(2,4-dichlorophenyl)-4,4-dimethyl-2-(1,2,4-triazol-1-yl)pentan-3-ol.

6. A process for preparing an enantiomer according to any one of the preceding claims, characterised in that (a) a mixture of said enantiomer with the other enantiomer of the compound of general formula (I) is resolved in known manner and the desired enantiomer is isolated in substantially optically pure form;

or (b) a compound of general formula (II)

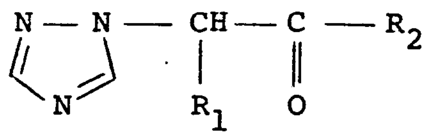

wherein $R_1$ and $R_2$ are as defined in claim 1, or salt or metal complex thereof, is stereospecifically reduced in known manner and the desired enantiomer is then isolated in substantially optically pure form.

7. A fungicidal or plant growth regulating composition characterised by comprising, as active ingredient, an enantiomer according to any one of claims 1 to 5 and a carrier or diluent for the active ingredient.

8. A method of combatting fungal diseases in, or regulating the growth of, a plant, characterised by comprising applying to the plant, to seed of the plant or to the locus of the plant or seed an enantiomer according to any one of claims 1 to 5.